# EUROPEAN PATENT SPECIFICATION

(11) **EP 2 531 262 B1**
(45) Date of publication and mention of the grant of the patent: **25.05.2016**
(21) Application number: 10702235.2
(22) Date of filing: 03.02.2010
(51) Int. Cl.: A61Q 11/00, A61K 8/06, A61K 8/20, A61K 8/24, A61K 8/55, A61K 8/86, A61K 8/92

(54) **ORAL CARE COMPOSITION COMPRISING A PRECURSOR OF CHLORINE DIOXIDE AND LIPOPHILIC EXCIPIENTS**
MUNDPFLEGEZUSAMMENSETZUNG MIT EINEM CHLORDIOXID-VORLÄUFER UND LIPOHILEN HILFSSTOFFEN
COMPOSITION D'HYGIÈNE BUCCALE CONTENANT UN PRÉCURSEUR DU DIOXYDE DE CHLORE ET DES EXCIPIENTS LIPOPHILES

(43) Date of publication of application: 12.12.2012
(73) Proprietor: Swissdent Cosmetics AG, 8001 Zürich (CH)
(72) Inventor: GHOSH, Sankha, CH-8001 Zürich (CH); VELKOBORSKY, Vaclav, CH-8008 Zürich (CH)
(74) Representative: Grimm, Siegfried
(86) International application number: PCT/CH2010/000029
(87) International publication number: WO 2011/094872

(56) References cited:
- WO-A1-99/43295
- WO-A2-03/022256
- WO-A2-2006/128269
- US-A- 5 283 056
- US-A1- 2006 045 855
- US-A1- 2008 247 972
- US-B2- 6 582 682

## Description

The invention relates to liquid oral care compositions, particularly in the form of a mouth spray or gurgling rinse, comprising non-ionic surfactants, flavoring oils, precursors of ClO2. The invention further relates to the manufacture and use thereof.

Chlorine dioxide (ClO2) is known in the field to function as bactericidal, bacteristatic, fungicidal, fungistatic, antimicrobial, antiseptic and antiviral as well as oxidizing agents (especially towards VSC's) even at very low concentrations, making it the most efficient and oral care application suitable agent towards reducing plaque count, halitosis and other related disadvantageous issues. Precursors thereof are typically used to formulate oral compositions. However, these precursors, due to their strong oxidizing property, are difficult to formulate commercial end-user products with, particularly, o/w microemulsions that possess (1) reasonable shelf-life (preferably more than 24 months), (2) stability of the ingredients used over the shelf-life, (3) stability of the composition over the shelf-life, (4) retention of quality of flavor, fragrance, taste and color, and (5) retention of packaging material.

Various oral care products are known for their ability to disrupt / prevent plaque formation, remove plaque, reduce and/or extinguish growth of microorganisms in plaque and to treat oral malodor, diseases and disorders, and for the attempts to incorporate them in commercial oral care products.

US 2008/247972 discloses an alcohol-free aqueous mouthwash comprising an oxidizing agent and a bactericide, for the prevention and treatment of halitosis. Specifically, the disclosed example provides 0.6% H2O2, 0.5% Polysorbate 20 and 0.15% flavors. The composition disclosed in this document does not form a stable isotropic clear o/w microemulsion. Further, the composition disclosed has a short shelf-life and instability in flavor.

US 6696047 discloses certain oral care compositions comprising chlorite and a chlorite stable flavor system, such as a dual phase rinse, for treatment of halitosis. Chlorite stable flavor systems of this document are substantially free of chlorite sensitive compounds that degrade in the presence of chlorite (see claim 1). Consequently, a number of known and well accepted flavors, such as Peppermint-, Wintergreen- or Eucalyptus oil, are less suited for this composition. Further, the presence of ethanol in a dual phase rinse (see ex. 4) is considered disadvantageous due to the fact that it dehydrates and desiccates oral mucosa, increasing cell desquamation and consequently increasing the formation of tongue coating and tonsilloliths, since proteolytic bacteria shall decompose these peeled off cells, originating volatile sulfur compounds (VSCs) in such process, as also being pointed out by US 2008/247972.

US 6582682 discloses certain oral care compositions in the form of a microemulsion comprising chlorite and ethoxylated hydrogenated castor oil, for treatment of halitosis. It has been found that none of these examples provided are stable for more than 3 weeks (stability test at 40°C), resulting (1) phase separation, (2) yellowing and (3) depletion in NaClO2 concentration by more than 90%. These effects may be ascribed by the fact that the micellar aggregates were not (1) stable enough towards leaking entrapped flavor / lipophilic substance from the micellar core, (2) micellar aggregates were not efficient enough to avoid confrontation between flavor phase and aqueous phase containing NaClO2, and (3) micellar aggregates may be too large which facilitates intermicellar coalescence within a short time during storage releasing entrapped flavoring agents to confront with aqueous NaClO2 and stipulating phase separation to the overall composition. Therefore the disclosed compositions in this prior art document are not stable and suitable in formulating practical and commercial oral-care products.

US5283056 discloses transparent oil-in-water microemulsions, free of oxidizing agents.

Consequently, it is an object of the present invention to provide improved compositions and to mitigate at least some of these drawbacks of the state of the art. In particular, it is an aim of the present invention to provide a composition that has a pronounced effect on oral hygiene and / or oral health, particularly halitosis, and improved shelf life.

These objectives are achieved by a composition as defined in claim 1. Further aspects of the invention are disclosed in the specification and independent claims, preferred embodiments are disclosed in the specification and the dependent claims.

The present invention will be described in more detail below. It is understood that the various embodiments, preferences and ranges may be combined at will. Further, depending on the specific embodiment, selected definitions, embodiments or ranges may not apply.

Unless otherwise stated, the following definitions shall apply in this specification:

The term "containing" shall, in the context of this invention, also include the meaning of comprising or consisting of.

The term "treatment of a disorder shall", in the context of this invention, also include the prevention and/or delay of progression of said disorder.

The term "microemulsion" is known in the field. It particularly denotes a thermodynamically stable isotropic dispersion of two immiscible liquids stabilized by a surfactant, often in combination with a co-surfactant. Such dispersion comprises homogeneously distributed droplets with diameter less than 100nm throughout a continuous phase making it clear and transparent or opalescent. An aqueous phase (which is typically the continuous phase) may contain dissolved electrolytes and any water-soluble species. The oil phase (which typically form the dispersed phase, droplets) may be a mixture of water-insoluble lipophilic substances. Although the microemulsions appear to be a clear transparent / opalescent mono-phase solution to human eye, they are actually a three-component (ternary) system, designated as oil / surfactant / water or water / surfactant / oil, wherein surfactant molecules form the interface through self-assembly with various possible structures of which micellar assembly is most frequent and common. There are two types of microemulsions of which one is "direct" wherein the oil, which is the minor component, is dispersed in water, which is the major component, designated by o/w. The second type is "reverse" / "inverse" wherein water, which is the minor component, is dispersed in the oil, which is the major component, designated by w/o.

The term "Micelle" is known in the field. It particularly denotes an aggregate / shelf-assembly (Mₙ) of surfactant molecules (nM) dispersed in a liquid colloid, where M denotes a surfactant molecule, often referred to as surfactant monomer, Mₙ denotes a micelle due to aggregation of n surfactant monomers and n is number of surfactant molecule/monomers. It is also known that there are two types of micelles: (1) Direct / typical micelle, designated by o/w, and (2) reverse / inverse micelle, denoted by w/o. The term "Co-surfactant" is known in the field. It particularly denotes the substance that collaborates with surfactants in reducing interfacial tension. In context to the present invention, a co-surfactant is either a mono- or a poly-ol species.

The term "Micellar migration" is known in the field. It particularly denotes the exchange of surfactant monomers, solubilized ingredients and fragment of micelle (fragment of surfactant aggregate that comprises surfactant monomers, solubilized ingredients and co-surfactant molecules) among the micelles.

The term "liquid oral solution composition" is known in the field. It particularly denotes a mouthwash, mouth spray, gurgling rinse and mouth rinse.

In a first aspect, the invention relates to a liquid oral care composition, in the form of a micro-emulsion, containing
a) one or more non-ionic surfactants, selected from the group consisting of polyoxyethylene sorbitan mono-esters of lauric acid, palmitic acid, stearic acid and oleic acid;
b) one or more lipophilic excipients, selected from the group consisting of Peppermint-, Spearmint-, Wintergreen- and Eucalyptus oil;
c) one or more water-soluble chlorine dioxide precursors, preferably selected from the group consisting of alkali metal chlorites, - chlorates and - hypochlorites,
d) one or more phosphate compound selected from the group consisting of alkali metal pyrophosphate;
e) water
f) optionally one or more sweeteners;
g) optionally one or more isotonic agents.

It was surprisingly found that the inventive compositions have a shelf-life of 36 months or longer at room temperature, do not suffer from yellowing discoloration, do not suffer from denaturation of flavor and taste and do not develop undesirable chlorine odor. Further, the inventive compositions retain 80% of sodium chlorite at the length of 36 months. Further, the inventive compositions show a pronounced effect on oral hygiene and / or oral health, particularly provide excellent results in controlling halitosis.

The invention is explained in further detail below:
**Microemulsion:** The inventive composition is typically present as an o/w microemulsion. In this microemulsion, the flavoring oils (constituent b) are in the micellar interior, surrounded by the surfactant (constituent a). The remaining constituents are present in the continuous aqueous phase. Non-ionic surfactants produce relatively larger o/w micelles than ionic surfactants, which are more spacious towards accommodating greater amount of lipophilic substances and more efficient in wrapping up solubilized molecules in their lipophilic core. Migration of surfactant monomers, solubilized additives/ingredients and micellar fragments among ionic micelles exclusively occur via intermicellar migration involving (1) exit from one micelle, followed by (2) diffusion in water phase and (3) association to another micelle. Migration mechanism involving intermicellar collisions (fusion-fission) is excluded due to strong repulsion potential amongst ionic micelles. Micellar collisions take place among the non-ionic micelles due to absence of intermicellar repulsive interaction. Intermicellar collision involves (1) temporary coalescence of two micelles during which exchange of surfactant monomers, solubilized additives and micellar fragments between the merged micelles take place, followed by (2) separation and re-formation. Micellar migration may take place for both ionic and non-ionic micelles via a third mechanism involving (1) loss of a micellar fragment that contains surfactant monomers, solubilized additives and co-surfactant, followed (2) diffusion in aqueous phase and (3) coagulation to other micelle. Solubilized additives are not embedded in such a fragment as good as in a full micelle, and therefore its diffusion through aqueous phase raises opportunity and probability of confrontation between solubilized lipophilic ingredients and the ingredients soluble in the aqueous phase. Confrontation between aqueous phase containing precursors of ClO2 and solubilized lipophilic agents (flavor / fragrance in particular) in micelles is severely disadvantageous, since it will reduce the stability of the ingredients of the composition. If the micelles are too large, the probability of intermicellar collision and as a result permanent coalescence releasing solubilized oil phase, increases. Therefore, surfactants as defined below are preferred in the context of this invention.
**Constituents:** It is understood that each constituent a) to g) may be present as one single component (e.g. a single flavoring oil) or as a combination of components (e.g. two or more flavoring oils). The constituents of the inventive composition are known per se (i.e. commercially available or available by known methods) although not combined as disclosed herein.
   **Constituent a)** Non-ionic surfactants are known in the field. For the inventive compositions, pharmaceutically acceptable liquid non-ionic surfactants having a mean HLB number between 10 and 20, preferably between 14 and 19.5, more preferably between 15 and 19 are suitable. Such surfactants include polyoxyethylene sorbitan mono-esters of lauric acid (Polysorbate 20), palmitic acid (Polysorbate 40), stearic acid (Polysorbate 60) and oleic acid (Polysorbate 80), respectively; such derivatives are known and commercially available (e.g. TEGO SML 20-80 supplied by Goldschmid and Tween 20-80 supplied by Croda), such as Polysorbate 20, Polysorbate 40, Polysorbate 60, Polysorbate 80. One or more surfactants, preferably one surfactant, may be employed in the inventive composition. Particularly good results are obtained when using Polysorbate 20 (e.g. supplied by Goldschmid under the trade name, TEGO SML 20) Suitable amounts of constituent a) are in the range of 15 - 30 wt.-%, preferably 18 - 22 wt.-%. The amount of surfactant used in the inventive composition is comparatively high. Without being bound to theory, it is believed that such high concentration improves stability of the composition and avoids the use of co-surfactants, as discussed below.
   **Constituent b)** One or more flavoring oils, are employed in the inventive composition.Such flavoring oils are selected from the group consisting of Peppermint oil, Spearmint oil, Wintergreen oil, Eucalyptus oil. Particularly good results are obtained when said flavoring oils are extracted from leaves of the corresponding plants. Such oils are commercially available as "extra pure" (e.g. supplied by Essencia, Winterthur, Switzerland). It was surprisingly found that flavoring oils of "extra pure" grade provided particularly good results. Without being bound to theory, it is believed that the absence of impurities in the flavoring oil stabilized the inventive composition, thereby providing an advantageous embodiment of the present invention. Suitable amounts of constituent b) are in the range of 0.2 - 8 wit.-%, preferably 0.5 - 5 wit.-%.
   **Constituent c)** One or more water-soluble chlorine dioxide releasing agents ("chlorine dioxide precursors") are employed in the inventive composition. Such chlorine dioxide releasing agents are known in the field and include alkali metal chlorites, - chlorates and - hypochlorites, preferably alkali metal chlorites and - chlorates, particularly preferably sodium chlorite, potassium chlorite, sodium chlorate and potassium chlorate. Advantageously, sodium chlorite is used. Suitable amounts of constituent c) are in the range of 0.01 - 0.5 wt.-%, preferably 0.1 - 0.5 wt.-%, particularly preferably 0.2 - 0.35 wt-%.
   **Constituent d)** One or more water-soluble phosphate compounds are employed in the inventive composition. Phosphate compounds are commonly used to regulate the pH and / or to provide an anti - tartar effect. Suitable phosphate compounds or combinations thereof, are known in the field and include alkali metal pyrophosphate. Advantageously, tetrasodium pyrophosphate is used. Suitable amounts of constituent d) are in the range of 0.1 - 2 wt.-%, preferably 0.5 - 1 wt.-%.
   **Constituent e)** The water employed may be any purity grade of water compliant with the intended use as an oral care product. Advantageously, de-ionized water is used. Suitable amounts of water are in the range of 60.05 - 84.5 wit.-%, preferably 70 - 80 wt.-%.
   **Constituent f)** One or more natural or artificial sweeteners may be added to the inventive composition. The addition of sweeteners is commonly done to improve consumer acceptance; any sweetener or combination of sweeteners known in the field may be used. Preferred are sweeteners that are stable towards oxidizing conditions and soluble in aqueous media, such as Acesulfame-K. Suitable amounts of constituent f) are in the range of 0 - 2 wt.-%, preferably 0.2 - 1 wt.-%.
   **Constituent g)** One or more isotonic agents may be added to the inventive composition. The addition of isotonic agents is commonly done to improve osmotic diffusion through rending the osmotic pressure of the composition close to that of oral mucosa; any isotonic agent or combination of isotonic agents known in the field may be used. It is understood that isotonic agents must be compatible with the intended use as oral care compositions, constituent g) may thus be more precisely defined as "oral isotonic agent". Preferred are isotonic agents that are stable towards oxidizing conditions and soluble in aqueous media, such as NaCl. Suitable amounts of constituent g) are in the range of 0 - 1 wt.-%, preferably 0.05 - 0.5 wt.-%.

Suitable formulations of the inventive composition are any liquid formulations known in the field. In an advantageous embodiment, the invention thus relates to a composition as described herein, which is an oral care composition, particularly a mouth spray, mouth wash, mouth rinse, gurgling rinse.

In one advantageous embodiment, the inventive composition is formulated as a **mouth spray.** Such formulations are suitable for cosmetic and therapeutic treatment, such as described below.

In a further advantageous embodiment, the inventive composition is formulated as a **gurgling rinse.** Such formulations are particularly suitable for the treatment of flu and bacterial infections to throat and larynx.

In an advantageous embodiment, the invention thus relates to an oral care composition as described herein, which is **free of,** or substantially free of **co-surfactants.** It was found that the inventive oral care composition is stable and transparent without addition of further co-surfactants. This is surprising considering the compositions disclosed in the prior art. Typically, co-surfactants, such as polyoles or mono-alcohols, e.g. glycol or ethanol, are used to improve stability of oral care formulations.

In an advantageous embodiment, the invention thus relates to an oral care composition as described herein, wherein **no additional bactericides** and / or no preservatives are present.

In an advantageous embodiment, the invention thus relates to an oral care composition as described herein, having a **pH** between 7 and 11, preferably between 7.5 and 9.5, much preferred between 8 and 9.

In an advantageous embodiment, the invention relates to an oral care composition as described herein, having a **viscosity** similar to water; typically in the range between 2.5 10⁻⁴ and 1.5 10⁻³ Pas.

In a second aspect, the invention relates to the use of a composition as described herein in cosmetic applications and / or therapeutic applications.

In one embodiment, the invention provides a composition as disclosed herein as pharmaceutical, particularly for the treatment, prevention and / or delay of progression of halitosis. The term "halitosis" is known in the field; it also includes oral malodor and bad breath. The compositions and methods of treatment of the present invention are particularly effective for treating or preventing halitosis. Halitosis may be caused from consumption of disadvantageous foods or drinks; from smoking. Halitosis may also be caused from high plaque count. Treatment may be effective irrespective of the underlying cause.

In one further embodiment, the invention provides a composition as disclosed herein as pharmaceutical, particularly for the treatment, prevention and / or delay of progression of diseases or conditions of the oral cavity. The term "diseases or conditions of the oral cavity" is known in the field; it particularly denotes periodontal disease, gingivitis, periodontitis, periodontosis, adult and juvenile periodontitis, and other inflammatory conditions of the tissues within the oral cavity, plus caries, necrotizing ulcerative gingivitis, and other conditions such as herpetic lesions, and infections that may develop following dental procedures such as osseous surgery, tooth extraction, periodontal flap surgery, dental implantation, and scaling and root planing. Also specifically included are dentoalveolar infections, dental abscesses (e.g., cellulitis of the jaw; osteomyelitis of the jaw), acute necrotizing ulcerative gingivitis (i.e., Vincent's infection), infectious stomatitis (i.e., acute inflammation of the buccal mucosa), and Noma (i.e., gangrenous stomatitis or cancrum oris). Oral and dental infections are more fully disclosed in Finegold, Anaerobic Bacteria in Human Diseases, chapter 4, pp 78-104, and chapter 6, pp 115-154 (Academic Press, Inc., NY, 1977). The compositions and methods of treatment of the present invention are particularly effective for treating or preventing periodontal disease (gingivitis and/or periodontitis) and resulting breath malodor. Further, the term "diseases or conditions of the oral cavity" covers systemic and local causes; it thus includes underlying diseases, disorders or malfunctions, such as periodontal diseases and disorders; tongue and throat diseases associated infections, viral infections (such as influenza), related bad-breath, shore throat problems, and bacterial infections to throat and larynx. Further, the term "disease or condition of the oral cavity" covers tooth whitening, tooth discoloration, tooth staining, tooth decay and tooth hypersensitivity.

In one further embodiment, the invention provides a composition as disclosed herein for use in the treatment, prevention and / or delay of progression of halitosis and / or diseases or conditions of the oral cavity.

In one further embodiment, the invention provides the use of composition as disclosed herein for the manufacturing of a pharmaceutical for the treatment, prevention and or delay of progression of halitosis and / or diseases or conditions of the oral cavity.

In one further embodiment, the invention provides the use of composition as disclosed herein for the cosmetic treatment of oral malodor.

In one further embodiment, the invention provides a method of cleaning the oral cavity of a subject in need thereof comprising the step of contacting the oral cavity with an effective amount of a composition as described herein.

The cosmetic and therapeutic effects of the composition as described herein are clearly different. While a cosmetic effect (such as treatment of halitosis) is directly achieved by one single application, a therapeutic effect (such as treatment of diseases of the oral cavity) is obtained by an application over a prolonged period, e.g. daily application over one week.

In a further advantageous embodiment, the cosmetic treatment comprises the step of applying once one or two doses of 0.1-1 ml, e.g. 0.25 ml, of the inventive composition.

In a further advantageous embodiment, the therapeutic treatment comprises the step of multiple applications of one or two doses of 0.1 - 1 ml, e.g. 0.25 ml, of a composition as described above. Such multiple applications include applications once a day, twice a day, or 3 times a day over a period of 2 or more days, such as 2 days, 5 days, 1 week, 2 weeks, 1 month.

In a third aspect, the invention relates to the manufacture of compositions as described herein. The manufacturing of microemulsions is known in the field and generally comprises the combination of the required constituents at room temperature or elevated temperatures using standard equipment.

In one embodiment, the invention relates to a method for manufacturing composition as described herein, comprising the steps of
a) providing a phase A, in the form of a microemulsion, containing constituents a), b) and water;
b) providing a phase B, in the form of a solution, containing constituents c), d), f), g) and water
c) combining said composition A and B to obtain a microemulsion.

In one embodiment, phase A is manufactured by providing a solution of constituents a) and b) and adding water to this solution under moderate agitation to obtain a microemulsion.

Depending on the constituents and the equipment used, the manufacturing takes place at 15 - 85 °C, preferably at room temperature.

The following examples further illustrate the invention without any limitation.

### Manufacturing Process:

A mixer was charged with Polysorbate. The oils were added with moderate agitation until a clear solution / micro-emulsion was obtained. Water was added slowly to the microemulsion with moderate agitation; the resulting mixture was agitated until a clear / transparent microemulsion was obtained: phase A.

In a separate container, water soluble additives ("electrolytes") were added to the water with moderate agitation and agitate until a clear aqueous solution was obtained: phase B.

Phase B was slowly added to phase A with high agitation. Agitation was continued until a transparent microemulsion was obtained.

| ex. | 1 wt.-% | 2 wt.-% | 3 wt.-% | 4 wt.-% | 5 wt.-% | 6 wt.-% |
|---|---|---|---|---|---|---|
| **phase A** | | | | | | |
| Polysorbate 20 TEGO SML20 | 20 | 20 | 20 | 20 | 20 | 20 |
| Mentha Piperita Oil | 2.8 | 1 | 2.8 | 1 | | |
| Eucalyptus Globulus Leaf oil | 1 | 0.1 | 1 | 0.1 | | |
| Gaultheria Procumbens oil (Wintergreen) | 0.5 | 0.1 | 0.5 | 0.1 | | |
| Spearmint oil | | | | | 0.5 | |
| Peppermint oil | | | | | | 0.5 |
| Deionized water | 30.5 | 33.6 | 30.5 | 33.6 | 34.3 | 34.3 |
| **phase B** | | | | | | |
| Deionized water | 43 | 43 | 43 | 43 | 43 | 43 |
| Tetrasodium pyrophosphate | 0.6 | 0.6 | 0.6 | 0.6 | 0.6 | 0.6 |
| NaClO2 (25%) | 1 | 1 | 1 | 1 | 1 | 1 |
| Acesulfame-K | 0.5 | 0.5 | 0.5 | 0.5 | 0.5 | 0.5 |
| NaCl | 0.1 | 0.1 | 0.1 | 0.1 | 0.1 | 0.1 |

### Stability Test

The compositions of ex. 1 - 6 possess 36 months of shelf-life. In particular, the compositions of ex. 1 - 6
(1) did not show phase separation in 36 months of storage at room temperature as well as three months of storage at 40°C;
(2) retained 99.5%, 95% and 90% of ClO₂ precursor in one, two and three years of storage at room temperature;
(3) retained 98%, 92% and 88% of ClO₂ precursor in one, two and three years of storage at 40°C and
(4) no development of malodor, mal-taste and mal-flavor did occur in 36 months of storage in room temperature as well as three months storage in 40°C.

Clinical Test:

The compositions of ex. 1 - 6 are suitable as a mouthspray.

A composition according to ex. 1 was clinically tested on more than 100 volunteers of different age and sex groups with halitosis problem, using a halimeter. Clinical study furnishes (1) instant extinction of bad-breath with longer than 5 hours of after-effect, (2) reduction of halitosis problem upon systematic intake of the composition over between one and three weeks, and (3) an improvement in whiteness and brightness to the tooth has been observed through "Spectroshade" study on the volunteers upon systematic use of the composition for a week. In addition, Oxyguard II was tested out on the volunteers, with positive outcome, after consumption of foods and drinks, which are known to cause bad breath, such as garlic enriched foods, beers, vodka.

Furthermore, it has been tested on the volunteers suffering from Flu and shore-throat problems due to bacterial infections by means of gurgling and rinsing; while an immediate relief was noticed, it beneficially improved their health within 3 days of systematic intake.

## Claims

1. A liquid oral care composition, in the form of a microemulsion, containing
(a) 15 - 30 wt.-% non-ionic surfactants selected from the group consisting of polyoxyethylene sorbitan mono-esters of lauric acid, palmitic acid, stearic acid and oleic acid;
(b) 0.2 - 8 wt.-% lipophilic excipients selected from the group consisting of Peppermint-, Spearmint-, Wintergreen-, Eucalyptus oil;
(c) 0.01 - 0.5 wt.-% of a water-soluble chlorine dioxide releasing agent,
(d) 0.1 - 2 wt.-% of a phosphate compound selected from the group consisting of alkali metal pyrophosphate;
(e) 60.05 - 84.5 wt.-% of water;
(f) 0 - 2 wt.-% of a sweetener;
(g) 0 - 1 wt.-% of an isotonic agent.

2. The composition according to claim 1, which is a mouth spray or gurgling rinse.

3. The composition according to any of claims 1 - 2, wherein no co-surfactants, particularly no alcohols, are present.

4. The composition according to any of claims 1 - 3, wherein no additional bactericides are present.

5. The composition according to any of claims 1 - 4, wherein no preservatives are present.

6. The composition according to any of claims 1 - 5, wherein
▪ said constituent a) is PEG 20 sorbitan monolaurate; and/or
▪ said flavoring oils b) are extracted from leaves; and/or
▪ said chlorine dioxide precursors c) being selected from the group consisting of potassium chlorite or sodium chlorite
▪ said phosphate compound d) is selected from the group consisting of tetrapotassium- and tetrasodium pyrophosphate; and/or
▪ said constituent e) is de-ionized water.

7. The composition according to any of claims 1 - 6, having a pH between 7 and 11.

8. The composition according to any of claims 1 - 6 having a shelf life of 36 months or more, in particular wherein 88% of component c) are retained during storage for 36 months at 40°C.

9. A method for manufacturing an oral care composition according to any of claims 1-8, comprising the steps of
(a) providing a phase A, in the form of a microemulsion, containing constituents a), b) and water;
(b) providing a phase B, in the form of a solution, containing constituents c), d), f), g) and water
(c) combining said composition A and B to obtain a microemulsion.

10. A composition according to any of claims 1 - 8 for use as pharmaceutical.

11. A composition according to any of claims 1 - 8 for use in the treatment, prevention and / or delay of progression (i) of disorders related to halitosis and (ii) of disorders or conditions of the oral cavity.

12. Use of a composition according to any of claims 1 - 8 for the cosmetic treatment of halitosis.

13. A method of cleaning the oral cavity of a subject in need thereof, comprising the step of contacting the oral cavity with an effective amount of a composition according to any of claims 1 to 8.

## Patentansprüche

1. Eine flüssige Mundpflegezusammensetzung in der Form einer Mikroemulsion, enthaltend
a) 15 - 30 Gew.% nichtionische Tenside, ausgewählt aus der Gruppe bestehend aus Polyoxyethylensorbitanmonoestern der Laurinsäure, Palmitinsäure, Stearinsäure und Ölsäure;
b) 0.2 - 8 Gew.% lipophile Hilfsstoffe, ausgewählt aus der Gruppe bestehend aus Pfefferminz-, Grüne Minze-, Wintergrün-, Eukalyptusöl;
c) 0.01 - 0.5 Gew.% eines wasserlöslichen Chlordioxid-abgebenden Mittels,
d) 0.1 - 2 Gew.% einer Phosphatverbindung, ausgewählt aus der Gruppe bestehend aus Alkalimetalpyrophosphat;
e) 60.05 - 84.5 Gew.% Wasser;
f) 0 - 2 Gew.% eines Süssstoffs;
g) 0 - 1 Gew.% eines isotonischen Mittels.

2. Die Mundpflegezusammensetzung nach Anspruch 1, welche ein Mundspray oder eine Gurgelspülung ist.

3. Die Mundpflegezusammensetzung nach einem der Ansprüche 1 - 2, wobei keine Co-Tenside, insbesondere keine Alkohole, vorhanden sind.

4. Die Mundpflegezusammensetzung nach einem der Ansprüche 1 - 3, wobei keine zusätzlichen Bakterizide vorhanden sind.

5. Die Mundpflegezusammensetzung nach einem der Ansprüche 1 - 4, wobei keine Konservierungsmittel vorhanden sind.

6. Die Mundpflegezusammensetzung nach einem der Ansprüche 1 - 5, wobei
- der Bestandteil a) PEG 20 Sorbitanmonolaurat ist; und/oder
- die Aromaöle b) aus Blättern extrahiert werden; und/oder
- die Chlordioxid-Grundstoffe c) aus der Gruppe bestehend aus Kaliumchlorid oder Sodiumchlorid ausgewählt werden
- der Phosphatbestandteil d) aus der Gruppe bestehend aus Tetrakalium- und Tetrasodiumpyrophosphat ausgewählt wird; und/oder
- der Bestandteil e) entionisiertes Wasser ist.

7. Die Mundpflegezusammensetzung nach einem der Ansprüche 1 - 6, wobei sie einen pH-Wert zwischen 7 und 11 hat.

8. Die Mundpflegezusammensetzung nach einem der Ansprüche 1 - 6, wobei sie eine Lebensdauer von 36 Monaten oder mehr hat, insbesondere wobei 88% des Bestandteils c) während einer 36 monatigen Lagerung bei 40°C zurückgehalten werden.

9. Ein Verfahren zur Herstellung einer Mundpflegezusammensetzung nach einem der Ansprüche 1 - 8, umfassend die Schritte
a) Bereitstellen einer Phase A in der Form einer Mikroemulsion, enthaltend die Bestandteile a), b) und Wasser;
b) Bereitstellen einer Phase B in der Form einer Lösung, enthaltend die Bestandteile c), d), f), g) und Wasser
c) Kombinieren der Zusammensetzung A und B um eine Mikroemulsion zu erhalten.

10. Eine Zusammensetzung nach einem der Ansprüche 1 - 8 zur Verwendung als Arzneimittel.

11. Eine Zusammensetzung nach einem der Ansprüche 1 - 8 zur Verwendung in der Behandlung, Vorsorge und/oder Verzögerung des Fortschreitens (i) von Halitose-bezogenen Erkrankungen und ii) von Erkrankungen oder Beschwerden der Mundhöhle.

12. Verwendung einer Zusammensetzung nach einem der Ansprüche 1 - 8 für die kosmetische Behandlung von Halitose.

13. Ein Verfahren zur Reinigung der Mundhöhle eines Subjekts der eine solche benötigt, umfassend den Schritt der Kontaktierung der Mundhöhle mit einer wirksamen Menge einer Zusammensetzung nach einem der Ansprüche 1 - 8.

## Revendications

1. Une composition de soin oral liquide, en forme de micro-émulsion, contenant
a) 15 - 30 % par poids des tensioactifs non-ioniques sélectionnés du groupe consistant de monoesters de sorbitan polyoxyéthyléné d'acide laurique, acide palmitique, acide stéarique et acide oléique;
b) 0.2 - 8 % par poids d'excipients lipophiles sélectionnés du groupe consistant d'huile essentielle de menthe, de menthe verte, de thé des bois, d'eucalyptus;
c) 0.01 - 0.5 % par poids d'un agent émettant du dioxyde de chlore hydrosoluble,
d) 0.1 - 2 % par poids d'un composé de phosphate sélectionnés du groupe consistant de pyrophosphate de métal alcalin;
e) 60.05 - 84.5 % par poids d'eau;
f) 0 - 2 % par poids d'un édulcorant;
g) 0 - 1 % par poids d'un agent isotonique.

2. La composition de soin oral selon la revendication 1, étant un spray buccal ou une rincezle de gargouillement.

3. La composition de soin oral selon l'une des revendications 1 - 2, aucun co-tensioactif, particulièrement aucun alcool, étant présent.

4. La composition de soin oral selon l'une des revendications 1 - 3, aucun bactéricide supplémentaire étant présent.

5. La composition de soin oral selon l'une des revendications 1 - 4, aucun conservateur étant présent.

6. La composition de soin oral selon l'une des revendications 1 - 5,
- ledit constituant a) étant du monolaurate de sorbitan PEG 20; et/ou
- lesdites huiles aromatisées b) étant extraites des feuilles; et/ou
- lesdits précurseurs de dioxyde de chlore c) étant sélectionnés du groupe consistant de chlorure de potassium ou chlorure de sodium
- ledit composé de phosphate d) étant sélectionné du groupe consistant de pyrophosphate tétrapotassique ou tétrasodique; et/ou
ledit constituent e) étant d'eau désionisée.

7. La composition de soin oral selon l'une des revendications 1 - 6, ayant un pH compris entre 7 et 11.

8. La composition de soin oral selon l'une des revendications 1 - 6, ayant une durée de vie de 36 mois ou plus, particulièrement 88% du composé c) étant gardé pendant le stockage pour 36 mois à 40°C.

9. Un procédé de fabrication d'une composition de soin oral selon l'une des revendications 1 - 8, comprenant les étapes de
a) prévoir une phase A, en forme d'une micro-émulsion, contenant les constituants a), b) et de l'eau;
b) prévoir une phase B, en forme d'une solution, contenant les constituants c), d), f), g) et de l'eau
c) combiner lesdites compositions A et B afin d'obtenir une micro-émulsion.

10. Une composition selon l'une des revendications 1 - 8 pour l'utilisation comme pharmaceutique.

11. Une composition selon l'une des revendications 1 - 8 pour l'utilisation dans le traitement, prévention et/ou retard d'une progression (i) de désordres liés à l'halitose et (ii) de désordres ou affections de la cavité orale.

12. Utilisation d'une composition selon l'une des revendications 1 - 8 pour le traitement cosmétique de l'halitose.

13. Un procédé de nettoyage de la cavité orale d'un sujet en ayant besoin, comprenant l'étape de contacter la cavité orale avec une quantité effective d'une composition selon l'une des revendications 1 - 8.
